(19) Europäisches Patentamt
European Patent Office
Office européen des brevets

(11) **EP 1 768 569 B1**

(12) **EUROPÄISCHE PATENTSCHRIFT**

(45) Veröffentlichungstag und Bekanntmachung des Hinweises auf die Patenterteilung:
**03.10.2007   Patentblatt 2007/40**

(21) Anmeldenummer: **05760059.5**

(22) Anmeldetag: **15.06.2005**

(51) Int Cl.:
*A61B 8/08* (2006.01)

(86) Internationale Anmeldenummer:
**PCT/DE2005/001068**

(87) Internationale Veröffentlichungsnummer:
**WO 2005/122905 (29.12.2005 Gazette 2005/52)**

(54) **VORRICHTUNG ZUR POSITIONIERUNG VON ULTRASCHALLWANDLERN ZUR BESTIMMUNG VON KNOCHENEIGENSCHAFTEN**

DEVICE FOR POSITIONING ULTRASONIC TRANSDUCERS IN ORDER TO DETERMINE BONE PROPERTIES

DISPOSITIF POUR PLACER DES TRANSDUCTEURS D'ULTRASONS POUR DETERMINER DES PROPRIETES OSSEUSES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **19.06.2004  DE 102004029900**

(43) Veröffentlichungstag der Anmeldung:
**04.04.2007   Patentblatt 2007/14**

(73) Patentinhaber:
- **Universitätsklinikum Schleswig-Holstein**
  **24105 Kiel (DE)**
- **CNRS**
  **75794 Paris Cedex 16 (FR)**
- **UNIVERSITE PIERRE ET MARIE CURIE**
  **75005 Paris (FR)**

(72) Erfinder:
- **BARKMANN, Reinhard**
  **24220 Flintbek (DE)**
- **GLÜER, Claus-C.**
  **22587 Hamburg (DE)**

- **LAUGIER, Pascal**
  **F-75017 Paris (FR)**
- **PADILLA, Frédéric**
  **F-75018 Paris (FR)**
- **MOSER, Urs**
  **CH-8006 Zürich (CH)**

(74) Vertreter: **Biehl, Christian et al**
**Boehmert & Boehmert,**
**Anwaltssozietät,**
**Niemannsweg 133**
**24105 Kiel (DE)**

(56) Entgegenhaltungen:
**US-A- 4 361 154          US-A- 6 077 224**

- **AKROUT L ET AL: "Feasibility of quantitative ultrasound measurement at the femur" 2002 IEEE ULTRASONICS SYMPOSIUM. PROCEEDINGS (CAT. NO.02CH37388) IEEE PISCATAWAY, NJ, USA, Bd. 2, 2002, Seiten 1353-1356 vol., XP002345072 ISBN: 0-7803-7582-3**

**Beschreibung**

Osteoporose ist eine systemische Skeletterkrankung, die durch eine niedrige Knochenmasse und Störung der Mikroarchitektur des Knochengewebes charakterisiert ist. Sie ist eine komplexe, multifaktorielle, chronische Erkrankung, die über Dekaden hinweg symptomlos verlaufen kann, bis der Knochenverlust eine Grenze erreicht, die im Folgenden zu einer erhöhten Knochenbrüchigkeit und Frakturanfälligkeit führt. Verschiedene Studien konnten zeigen, dass mit der Erniedrigung der Knochendichte das Risiko für Frakturen steigt.

Die klinische Osteoporosediagnostik umfasst mehrere Schritte und hat zum Ziel, ein individuelles Risikoprofil zu erstellen, woran sich die Therapieentscheidung orientiert. Bei der nichtinvasiven Bestimmung der Knochendichte sind grundsätzlich zwei methodische Ansätze zu unterscheiden - die Messung mit ionisierender Strahlung und die akustischen Messverfahren mit Ultraschall. Bei den Messverfahren mit ionisierender Strahlung wird der Knochenmineralsalzgehalt mittels Single- oder Dual-Photonen-Absorptionsmetrie (SPA oder DPA), Dual-Röntgen-Absorptionsmetrie (DXA) oder Quantitativer Computertomographie (QCT) bestimmt. Diese Verfahren messen mittels ionisierender Strahlung den Mineralsalzgehalt im Knochen, erkennen aber nicht seine mechanischen Qualitäten.

Quantitativer Ultraschall (QUS) stellt eine Alternative zur Röntgenstrahldensitometrie dar. Mittels Ultraschall kann die frequenzabhängige Schallabschwächung (BUA) und die Ultraschalltransmissionsgeschwindigkeit (SOS) im Knochen ermittelt werden. Da diese Parameter mit den mechanischen und strukturellen Eigenschaften des Knochens korrelieren, eignen sich Ultraschalltechniken dazu, die Elastizität, Geometrie und Struktur des Knochens darzustellen und so das Risiko für osteoporotische Frakturen einzuschätzen. Die Vorteile dieses Verfahrens liegen in der fehlenden Strahlenbelastung, den geringen Kosten, der einfachen und schnellen Messung und der universellen Einsetzbarkeit der Geräte.

Die derzeit kommerziell erhältlichen Ultraschall-Systeme (z.B. Achilles Insight, Fa. GE-Lunar; DTU-one, Fa. OSI/Osteometer; QUS-2, Fa. Metra/Quidel) wurden für Messungen der Knochendichte an einigen peripheren Messorten, hauptsächlich am Kalkaneus entwickelt. Dieser wurde im Rahmen radiologischer Knochendichteverfahren gut validiert, ist einfach zu erreichen und ein gewichtstragender Knochen, der einen hohen Anteil an trabekulären Strukturen besitzt.

Die bekannten Geräte bieten verschiedene Modifikationen für den Einsatz der Wandler am Calcaneus oder den Phalangen und unterschiedliche Ansätze der Signalauswertung.

Aus der US 4 361 154 ist ein Ultraschallgerät mit den Merkmalen des Oberbegriffs des Anspruch 1 bekannt. Die US 3 847 141 offenbart eine Vorrichtung zum Messen der Dichte einer Knochenstruktur, wie eines Fingerknochens oder Fersenknochens, um deren Kalziumgehalt zu überwachen. Diese Vorrichtung umfasst ein Paar einander gegenüberliegender Ultraschallwandler, die innerhalb einer Klemmvorrichtung gehalten werden, die an dem zu analysierenden Knochen fixiert ist. Die US 4 457 311 betrifft ein System zur Durchführung einer Ultraschallabtastung eines menschlichen Rückens unter Verwendung einer Anordnung aus Ultraschall-Wandlerelementen. Insbesondere kann die Wirbelsäule abgebildet werden, indem die Laufzeit von Ultraschallimpulsen gemessen wird. Der Vorrichtung fehlt jedoch jegliches Mittel zum automatischen Auswählen eines Punktes der Anordnung, um einen geeigneten Bereich von Interesse zu identifizieren, wenn die Vorrichtung erneut auf dem Patienten angeordnet wird. In der US 5 840 029 wird ein Ultraschall-Dichtemessgerät beschrieben, das eine Anordnung von Wandlerelementen aufweist. Die Auswertung der Signale erfolgt hier, um die "region of interest" wird während des Scanprozesses zu lokalisieren. Die US 6 135 964 offenbart ein Ultraschalldensitometer mit einer Einrichtung zur Sicherung der Signalqualität. Die Erfindung beschreibt ein Verfahren zur wiederholbaren Positionierung und Ankopplung der Ultraschallwandler an den Fuß, welches ausschließlich an der Ferse Anwendung findet. Das rückgestreute Ultraschallsignal wird hierbei als feedback für die automatische Positionierung genutzt.

Ultraschallverfahren finden bisher nur an einigen peripheren Messorten, hauptsächlich dem Kalkaneus, Anwendung. Sie gestatten zwar die Abschätzung eines osteoporotischen Frakturrisikos am Skelett (z.B. Femur, Wirbelkörper und Unterarm), das Frakturrisiko des Femurs lässt sich jedoch deutlich besser durch eine Messung direkt an diesem Ort abschätzen. Anders als am Kalkaneus lässt sich die Position und Ausrichtung des Femurs aber nicht durch äußere Fixierungsmaßnahmen festlegen. Die genaue Interpretation der Messergebnisse zur Bewertung der Knocheneigenschaften aus dem Ultraschallsignal und die Standardisierung des Verfahrens erfordert jedoch, konsistente Regionen in jedem Knochen wiederzufinden. Wiederholungsmessungen setzen eine hohe Präzision (Wiederholungsgenauigkeit) im Auffinden der Messregion und der Richtung relativ zu den Wandlern voraus.

Eine Erhöhung der Genauigkeit setzt das Festlegen einer Referenzebene und die exakte Positionierung der Ultraschallwandler in Bezug zu dieser Referenz voraus. Dieses lässt sich erreichen, wenn die zu interessierende Region aus einem gut definierten Winkel durchschallt wird, bzw. das aus dem Knochen heraus zurückgestreute Signal aus dem möglichst gleichen Winkel gemessen wird.

Es ist die Aufgabe der Erfindung, eine Vorrichtung zu schaffen, die die präzise Positionierung von Ultraschallwandlern zur Transmission von Ultraschallstrahlung durch Knochengewebe in vivo relativ zu einer durch die Lage des Knochens, insbesondere des Femurs, definierten Referenzebene in der Weise erlaubt, dass Ultraschallmessungen mit hoher Reproduzierbarkeit und auch möglichst guter inter-individueller Vergleichbarkeit ermöglicht werden.

Die Aufgabe wird gelöst durch eine Vorrichtung mit den Merkmalen des Anspruchs 1. Die Unteransprüche geben vorteilhafte Ausgestaltungen an.

Die erfindungsgemäße Vorrichtung umfasst ein Ultraschallgerät zur Bestimmung von Knocheneigenschaften mit wenigstens zwei Ultraschallwandlern, einer Auswerteeinheit und einer Positioniereinheit, die die paarweise aufeinander ausgerichteten Ultraschallwandler in einer Ebene verschiebbar und verkippbar führt. Des Weiteren ermöglicht eine Recheneinheit mindestens drei Punkte auf der Knochenoberfläche aus den vom Knochen reflektierten Ultraschall-Signalen zu ermitteln und daraus eine Referenzebene zu berechen. Die Wandler können für eine anschließende Messung in relativ zu der errechneten Referenzebene definierte Positionen gefahren werden.

Die Erfindung wird im Folgenden anhand eines möglichen Ausführungsbeispiels beschrieben.

Zur Erläuterung dieses Beispiels dienen folgende Abbildungen:

Fig. 1 zeigt ein Ausführungsbeispiel der erfindungsgemäßen Vorrichtung mit Angaben zu den vorgesehenen Bewegungsmöglichkeiten,

Fig. 2 zeigt eine Prinzipdarstellung der Kapselung mit flexiblem Andruck.

Die Vorrichtung umfasst wenigstens zwei gegenüberliegende Ultraschallwandler (T1, T2), die aufeinander zu ausgerichtet und diesbezüglich durch Verbindung über eine starre Gabel fixiert sind. Die Erfindung sieht vor, dass die Gabel durch einen elektronisch ansteuerbaren Antrieb entlang ihrer Symmetrieebene gezielt verschoben werden kann.

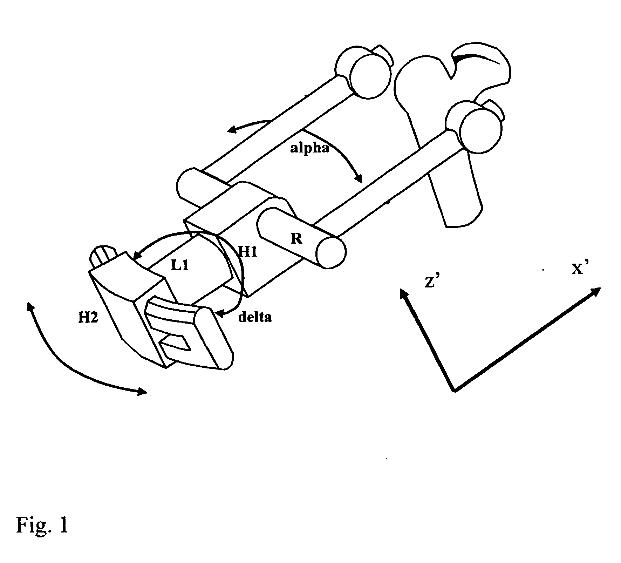

In Fig. 1 besteht die Gabel aus einem starren Rohr (R) mit fest verbundenen Seitenarmen (R1, R2). Die Gabel kann in ihrer Halterung H1 um den Winkel alpha gedreht werden, womit eine Bewegung der Wandler auf einem Kreisbogen in der Scanebene (hier: x'-z'-Ebene) stattfindet. Diese Drehbewegung verfährt die Wandler hauptsächlich in z'-Richtung. Mit dem verstellbaren Lager L1 kann die Halterung H1 zusätzlich in x'-Richtung verschoben werden.

Selbstverständlich sind andere Ausbildungen des Translationsantriebs der Gabel denkbar. Wesentlich ist, dass eine genau gesteuerte, die Orientierung der Gabel erhaltende Verschiebung parallel zur Symmetrieebene der Gabel möglich ist.

Erfindungsgemäß wird diese Symmetrieebene mit einer vom zu untersuchenden Knochen definierten Referenzebene zur Deckung gebracht, bevor durch Translation der Gabel unter Aussendung von Ultraschall der eigentliche Scan erfolgt. Es ist natürlich auch möglich, die Gabel zunächst an der Referenzebene auszurichten, anschließend um definierte Winkel zu verdrehen und den Scan entlang einer beliebig verkippten Ebene durchzuführen. Eine weiterführende Programmsteuerung würde ferner gestatten, den Scan sogar an einer gekrümmten Fläche entlang zu führen, wenn der Translationsantrieb der Gabel simultan mit den Stellantrieben der Gabeldrehgelenke angesteuert wird. Alle Vorgehensweisen setzen für die Reproduzierbarkeit aber eine präzise Initialorientierung der Gabel an der Referenzebene des Knochens voraus. Wie diese zu erreichen ist, ist zentraler Gegenstand der vorliegenden Erfindung.

Bei der erfindungsgemäßen Vorrichtung ist die Gabel drehbar um wenigstens zwei Achsen im Raum ausgebildet, wobei die Verdrehung über ansteuerbare Stellantriebe, vorzugsweise Schrittmotoren, erfolgt.

In Fig. 1 geschieht die Justierung in den beiden Winkeln beispielsweise durch eine Drehung des Lagers L1 um den Winkel delta und durch eine Verschiebung der Halterung H2 entlang der Schiene L2. Die Schiene L2 ist kreisbogenförmig ausgebildet, so dass die Bewegung der Halterung H2 eine gleichzeitige Drehung um den Winkel beta bewirkt (Goniometerrührung). Auch hier sind natürlich andere Varianten der Ausgestaltung denkbar, um die Gabel um zwei unabhängige Winkel zu verdrehen. Es ist im Übrigen vorteilhaft, Näherungssensoren zum Auffinden einer definierten Anfangsposition der Gabel einzusetzen, was zum Stand der Technik zählt.

Die optimale Referenzebene wird erfindungsgemäß durch eine Mittenfläche durch den Knochenschaft definiert. Im Falle des proximalen Femurs wird diese besonders bevorzugt durch die Mittenachse durch den Schaft und die Mittenachse durch den Hals aufgespannt.

Die Erfindung sieht vor, diese Referenzebene mittels Ultraschallreflektion zu ermitteln und die Gabel mit den Wandlern zunächst automatisch senkrecht zu dieser Ebene auszurichten. Die Prozedur umfasst dabei drei Phasen.

1. Durch beidseitiges Abscannen des Knochens mittels Ultraschallreflektion an n frei wählbaren Punkten werden zunächst die Schalllaufzeiten zwischen den Wandlern ($T_1$, $T_2$) und Knochenoberfläche gemessen. Die Wandler können dabei jeweils als Sender und Empfänger von Ultraschallstrahlung fungieren. Insbesondere können die Wandler Ultraschallsignale aussenden und reflektierte Signale von der Knochenoberfläche empfangen. Aus den messbaren Signallaufzeiten lassen sich die Abstände jedes Wandlers zu den gewählten Punkten auf der ihm zugewandten Knochenoberfläche berechnen. Die Abstände der Wandler $a_1,..., a_n$ für $T_1$ und $b_1,..., b_n$ für $T_2$ zur Knochenoberfäche bilden die Komponenten der n-dimensionalen Vektoren

$$\vec{A} = \begin{pmatrix} a_1 \\ \vdots \\ a_n \end{pmatrix} \quad \text{und} \quad \vec{B} = \begin{pmatrix} b_1 \\ \vdots \\ b_n \end{pmatrix}$$

2. Die günstige Wahl der Referenzebene ist abhängig von der Form und Dicke des Knochens, seiner Struktur und der interessierenden Meßregion (a priori Wissen). Insbesondere ist es notwendig, die Abstände der gewünschten Referenzebene beiderseits zur Knochenoberfläche (k1, k2) abschätzen zu können. Mit Hilfe dieser Werte und dem bekannten Gesamtabstand (W) der Wandler (T1, T2) lassen sich zwei n x n-Koeffizientenmatrizen ($K^A$ $K^B$) festlegen vorzugsweise Diagonalmatrizen, deren Matrixkomponenten die präzise Ausrichtung der Wandler zur Referenzebene ermöglichen. Die Abstände der Referenzebene zur Knochenoberfläche (k1, k2) werden als a priori Wissen vorausgesetzt. Desweiteren ist der Abstand (W) beider Wandler technisch bedingt und damit ebenfalls bekannt. Der Abstand der Wandler zur Knochenoberfläche (a1, b1) wird mittels Reflektionsmessung ermittelt. Erfindungsgemäß ist es das Ziel der Positionierung die Referenzebene mit der Mittenebene der Wandler in Deckung zu bringen.

3. Das Verdrehen der Gabel mit den Wandlern um die Winkel beta und delta ermöglicht es, die Wandler so zur Knochenoberfläche auszurichten, dass die Symmetrieebene mit der definierten Referenzebene vorzugsweise innerhalb des Knochens parallel liegt und sich vorzugsweise deckt. Ein möglicher Algorithmus zur Bestimmung der Stellwinkel für die Gabel besteht darin, den Differenzvektor

$$\vec{D} = \underline{K}^A \vec{A} - \underline{K}^B \vec{B}$$

zu berechnen und bezüglich einer Norm im n-dimensionalen Raum, vorzugsweise der euklidischen Norm, zu minimieren. Die Minimierung erfolgt automatisch durch Variation der Stellwinkel die die Abstände der Wandler vom Knochen bestimmen. Die konstanten Koeffizientenmatrizen $\underline{K}^A$ und $\underline{K}^B$ sind vom Anwender seinem a priori Wissen entspechend vorzugeben.

$$\left(\underline{K}^B\right)_{i,j} = \frac{W}{W - 2k'_i}$$

für i=1,...n, j=1,...,n und i=j und 0 sonst

$$\left(\underline{K}^A\right)_{i,j} = \frac{W}{W - 2k_i}$$

für i=1,...n, j=1,...,n und i=j und 0 sonst

Es ist eine bevorzugte Ausgestaltung der Erfindung, anhand eines Transmissionsbildes des Knochens die zu untersuchenden Punkte zu wählen. Zur leichteren Reproduzierbarkeit der Messungen ist es sinnvoll anatomisch charakteristische Punkte auszusuchen, die bei allen Patienten leicht aufzufinden sind. Die Koordinaten der Punkte in der Bildebene lassen sich mit einer Bildverarbeitungssoftware nach dem Stand der Technik automatisch ermitteln. Diese Punkte definieren zugleich drei Linien senkrecht zur Bildebene. Diese Linien durchstoßen die Oberfläche des Knochens in je zwei Punkten, jeweils auf einer Seite des Knochens, die einem der Wandler gegenüberliegt.

Das Aufsuchen der günstigsten Winkel beta und delta ist mit herkömmlichen Optimierungsprozeduren (z.B. Gradientenabstieg etc.) im Rechner vollautomatisch möglich. Bekanntlich sind andere Minimierungsaufgaben anstelle des Minimums der Norm des Differenzvektors geeignet, die gewünschte Minimierung der Abstandsdifferenzen zu erzielen.

Der Computer stellt die als optimal berechneten Winkel durch Ansteuerung der Stellantriebe ohne Zutun des Bedieners ein. Während des anschließenden Scanvorgangs werden sie entweder gar nicht mehr oder einem vorgegebenen Programm folgend geändert. Bei einer Wiederholung der Messung am selben Patienten zu einem späteren Zeitpunkt stellt die Vorrichtung sicher, dass die Messung erneut über exakt dieselbe Scanfläche erfolgt.

Es ist eine bevorzugte Ausgestaltung der Erfindung, besondere Maßnahmen zur Einkopplung des Schallstrahls in den Körper zu ergreifen. Zur Minderung des Impedanzkontrastes zwischen Weichgewebe und Schallgeber wird die Benutzung einer Flüssigkeit, z.B. Öl, vorgeschlagen. Um die Flüssigkeit einzuschließen, sind bevorzugt flexible Gefäße zwischen Wandlern und Hautoberfläche vorgesehen, welche sich an verschiedene Körperrundungen und Körperdicken anpassen können.

Um eine genaue und präzise Messung der Schallgeschwindigkeit zu ermöglichen, ist die Konstanz des Wandlerabstandes entscheidend. Daher dürfen die Gefäße mit der Koppelflüssigkeit nicht rückseitig von den Wandlern oder dem Wandlerbügel abgestützt werden, da dann Änderungen des Wandlerabstandes auftreten.

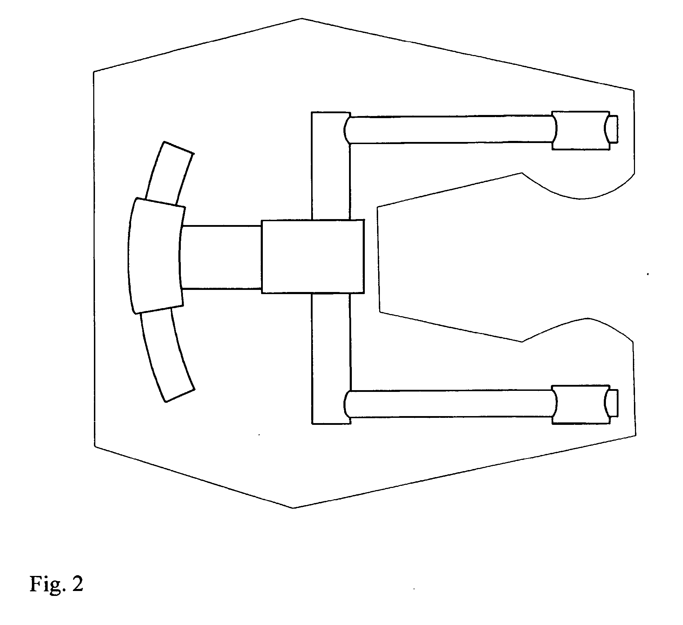

In Fig. 2 ist ein vorteilhaftes Design dargestellt, bei dem sich die komplette Scanmechanik im Flüssigkeitsbad befindet. Das betrifft auch die Antriebsmotoren, die entweder gekapselt sein müssen, oder in denen die beweglichen Teile ebenfalls im Ölbad laufen. Letzteres ist z.B. bei kontaktlosen Motoren wie Schrittmotoren möglich. Fig. 2 zeigt die prinzipielle Anordnung dieser Kapselung. Dünn gezeichnet sind flexible Membranen (Latex, Polyurethan o. ä.). Durch Druckerhöhung in der Flüssigkeit werden diese gegen die Haut gedrückt. Diese Druckerhöhung kann z.B. durch eine kleine Membranpumpe bewerkstelligt werden, die Flüssigkeit zwischen dem inneren Behälter und einem Ausgleichsbehälter hin- und herpumpen kann. Um die akustischen Eigenschaften konstant zu halten, wird die Koppelflüssigkeit auf konstanter Temperatur gehalten, bevorzugt auf Körpertemperatur.

**Patentansprüche**

1. Ultraschallgerät zur Bestimmung von Eigenschaften eines zu untersuchenden Knochens mit wenigstes zwei Ultraschallwandlern, einer Auswerteeinheit und eine Positioniereinheit, die wenigstens zwei Ultraschallwandler paarweise aufeinander ausgerichtet in einer Ebene verschiebbar und verkippbar führt, **gekennzeichnet durch** eine Recheneinheit, die aus den von dem Knochen reflektierten Ultraschall-Signalen mindestens drei Punkte auf der Knochenoberfläche ermittelt, eine Referenzebene im Knochen berechnet und die wenigstens zwei Wandler für eine anschließende Messung in relativ zu der errechneten Referenzebene definierte Positionen fährt.

2. Vorrichtung nach Anspruch 1, **dadurch gekennzeichnet, dass** die Recheinheit einen durch Verkippen der Wandler minimierten positiven Zahlenwert ermittelt, der die Norm eines n-dimensionalen Vektors ist, wobei n die Anzahl der vorgegebenen Positionen ist, zu denen die Halterung durch den ersten Stellantrieb bewegt wird.

3. Vorrichtung nach Anspruch 2, **dadurch gekennzeichnet, dass** der Vektor gemäß $\vec{D} = \underline{K}^A\overline{A}-\underline{K}^B\overline{B}$ berechnet wird, wobei $\underline{K}^A$ und $\underline{K}^B$ nxn-Matrizen mit konstanten Koeffizienten und die i-ten Komponenten von $\vec{A}$ und $\vec{B}$ die gemessenen Abstände je eines Wandlers zur Knochenoberfläche an der i-ten Position der Halterung sind.

4. Vorrichtung nach Anspruch 3, **dadurch gekennzeichnet, dass** die Komponenten der nxn-Matrizen aus a priori Wissen über Knochenmorphologie und Knochenanatomie bestimmt sind.

**Claims**

1. Ultrasound apparatus for the determination of the properties of the bone to be investigated with at least two ultrasound transducers, one assessment unit and one positioning unit, which drives at least two ultrasound transducers arranged in coupling with each other on a movable and inclinable plane **characterized by** a calculating unit which ascertains at least three points on the surface of the bone, calculates a reference plane in the bone and shifts the at least two transducers for a supplementary measurement relative to the calculated reference plane-defined positions

2. Device according to Claim 1, **characterized** thereby, that the calculating unit by means of tilting of the transducers ascertains the minimized positive numerical amount which is the norm of an n-dimensional vector by which n is the number of the prescribed positions to which the mounting is moved by the first positioning drive..

3. Device according to Claim 2, **characterized** thereby, that the vector calculated according to $\vec{D} = \underline{K}^A\overline{A}-\underline{K}^B\overline{B}$, by which $\underline{K}^A$ and $\underline{K}^B$ n x n matrices with constant coefficients and the i-th components of $\overline{A}$ and $\overline{B}$ are the measured distances of each one of the transducers to the bone surface at the i-th positions of the mounting.

4. Device according to Claim 3, **characterized** thereby, that the components of the n x n matrices are determined from a priori knowledge about bone morphology and bone anatomy..

**Revendications**

1. Appareil à ultrasons pour déterminer des propriétés d'un os à examiner, comprenant au moins deux transducteurs ultrasonores, une unité d'évaluation et une unité de positionnement qui guide au moins deux transducteurs ultrasonores alignés l'un sur l'autre par paires dans un plan avec possibilité de translation et de basculement, **caractérisé par** une unité de calcul qui détermine à partir des signaux ultrasonores réfléchis par l'os au moins trois points à la surface de l'os, calcule un plan de référence dans l'os et amène lesdits au moins deux transducteurs ultrasonores, pour une mesure suivante, dans des positions définies par rapport au plan de référence calculé.

2. Dispositif selon la revendication 1, **caractérisé en ce que** l'unité de calcul détermine une valeur numérique positive minimisée par basculement des transducteurs qui est la norme d'un vecteur à n dimensions, n étant le nombre des positions prédéfinies vers lesquelles le support est déplacé par le premier actionneur de positionnement.

**3.** Dispositif selon la revendication 2, **caractérisé en ce que** le vecteur est calculé selon $\vec{D} = \underline{K}^A \overline{A} - \underline{K}^B \overline{B}$, $\underline{K}^A$ et $\underline{K}^B$ étant des matrices nxn à coefficients constants et les $i^{èmes}$ composantes de $\overline{A}$ et $\overline{B}$ les distances mesurées de chaque fois un transducteur par rapport à la surface de l'os à la $i^{ème}$ position du support.

**4.** Dispositif selon la revendication 3, **caractérisé en ce que** les composantes des matrices nxn sont déterminées à partir de connaissances a priori sur la morphologie de l'os et l'anatomie de l'os.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4361154 A
- US 3847141 A
- US 4457311 A
- US 5840029 A
- US 6135964 A